# EUROPEAN PATENT SPECIFICATION

(11) **EP 1 094 115 B1**
(45) Date of publication and mention of the grant of the patent: **06.05.2009**
(21) Application number: 99926878.2
(22) Date of filing: 01.07.1999
(51) Int. Cl.: C12N 15/86, A61K 48/00, A01K 67/027, C12N 7/00

(54) **(-)-STRAND RNA VIRUS VECTOR FOR NERVE CELLS**
(-)-STRANG RNS VIRUSVEKTOR FÜR NERVENZELLEN
VECTEUR DE VIRUS A ARN A BRIN NEGATIF POUR DES CELLULLES NERVEUSES

(30) Priority: 03.07.1998 JP 20433398
(43) Date of publication of application: 25.04.2001
(73) Proprietor: Dnavec Research Inc., Tsukuba-shi, Ibaraki 305-0856 (JP)
(72) Inventor: FUKUMURA, Masayuki, DNAVEC Research Inc., Tsukuba-shi, Ibaraki 305-0856 (JP); ASAKAWA, Makoto, 319 Shionogi-Kanzakigawa-Ryo, Toyonaka-shi, Osaka 561-0825 (JP); HASEGAWA, Mamoru, DNAVEC Research Inc., Tsukuba-shi, Ibaraki 305-0856 (JP)
(74) Representative: Straus, Alexander
(86) International application number: PCT/JP1999/003552
(87) International publication number: WO 2000/001837

(56) References cited:
- EP-A- 0 863 202
- EP-A- 1 186 667
- ATSUSHI KATO ET AL: "INITIATION OF SENDAI VIRUS MULTIPLICATION FROM TRANSFECTED CDNA OR RNA WITH NEGATIVE OR POSITIVE SENSE" GENES TO CELLS, OXFORD, GB, vol. 1, no. 6, June 1996 (1996-06), pages 569-579, XP002911572 ISSN: 1356-9597
- MORIYA C ET AL: "LARGE QUANTITY PRODUCTION WITH EXTREME CONVENIENCE OF HUMAN SDF-1ALPHA AND SDF-1BETA BY A SENDAI VIRUS VECTOR" FEBS LETTERS, ELSEVIER SCIENCE PUBLISHERS, AMSTERDAM, NL, vol. 425, no. 425, 20 March 1998 (1998-03-20), pages 105-111, XP000887284 ISSN: 0014-5793
- DATABASE BIOSIS [Online] BIOSCIENCES INFORMATION SERVICE, PHILADELPHIA, PA, US; 1997, SUZUKI JUN-ICHI ET AL: "Prevention of graft coronary arteriosclerosis by antisense cdk2 kinase oligonucleotide" XP002343324 Database accession no. PREV199799702925 & NATURE MEDICINE, vol. 3, no. 8, 1997, pages 900-903, ISSN: 1078-8956
- DATABASE BIOSIS [Online] BIOSCIENCES INFORMATION SERVICE, PHILADELPHIA, PA, US; 1996, YAMADA KAZUO ET AL: "Efficient oligonucleotide delivery using the HVJ-liposome method in the central nervous system" XP002343325 Database accession no. PREV199799322964 & AMERICAN JOURNAL OF PHYSIOLOGY, vol. 271, no. 5 PART 2, 1996, pages R1212-R1220, ISSN: 0002-9513
- DATABASE BIOSIS [Online] BIOSCIENCES INFORMATION SERVICE, PHILADELPHIA, PA, US; 1994, DE FIEBRE CHRISTOPHER M ET AL: "Differential adenoassociated virus vector-driven expression of a neuropeptide Y gene in primary rat brain astroglial cultures after transfection with Sendai virosomes versus Lipofectin" XP002343326 Database accession no. PREV199497312728 & NEUROCHEMICAL RESEARCH, vol. 19, no. 6, 1994, pages 643-648, ISSN: 0364-3190
- NEUROCHEMICAL RESEARCH, Vol. 18, No. 10, (1993), CHRISTOPHER M. DE FIEBRE et al., "Fusogenic Properties of Sendai Virosome Envelopes in Rat Brain Preparations", pages 1089-1094, XP002922225.
- JOURNAL OF CONTROLLED RELEASE, Vol. 54, No. 1, (1998), MAHITO NAKANISHI et al., "Gene Transfer Vectors Based on Sendai Virus", pages 61-68, XP002922226.

## Description

### Technical Field

The present invention relates to an in vitro method of transferring a gene for gene therapy of nerve cells using a virus vector, more specifically, a negative-sense RNA virus vector, and related subject matter.

### Background Art

It is an extremely important object in the gene therapy for humans and animals to develop a system whereby a gene is transferred into target organs and target cells with a high efficiency. Methods for transferring a gene include the calcium phosphate method, DEAE-dextran method, cationic liposome method, electroporation method, etc., and especially methods for transferring a gene *in vivo* include a method using virus or liposome, or a direct transfer method. Among them, the gene transfer performed using "a virus vector" obtained by recombination of viral gene is extremely useful for the transfer of a gene into cells, for example, for gene therapy because of easy transfer procedure and its high transfer efficiency.

Virus vectors commonly used at present in gene therapy include retrovirus vector, herpes simplex virus (HSV) vector, adenovirus vector, and adeno-associated virus (AAV) vector, etc. In particular, along with the recent progress in analysis of brain functions using MRI and PET, there has been an increased demand for vectors capable of efficiently infecting non-dividing nerve cells and mediating a high level transgene expression in the infected cells. Therefore, adenoviral vector, herpes simplex viral vector, AAV, HIV, etc. have received considerable attention.

Although HSV has been reported to be capable of transferring a gene into ganglions in the peripheral nervous system, a problem remains on the amount of its expression (Gene Therapy, 1995, 2: 209-217). HIV infection of nerve cells has also been confirmed (Nature Biotechnology, 1997, 15: 871-875). Since the chromosomal position into which the HIV genome is inserted is hardly predictable, there are possibilities of damaging a normal gene, activating a cancer gene, and inducing excessive or suppressed expression of a desired gene.

AAV has been used for the brain treatment in Parkinson's disease (Exp. Neurol., 1997, 144: 147-156) and mucopolysaccharidosis type VII (ASGT meeting, 1998, Abstract No. 692). However, there have been reported an incomplete transfer of the introduced gene into the substantia nigra in Parkinson's disease and its insufficient expression in the brain in mucopolysccharidosis type VII.

Adenovirus has been most commonly used at present, and reported to be capable of transferring a gene into the pyramidal cell layer of hippocampus (Nature Medicine, 1997, 3: 997-1004). However, adenovirus has drawbacks, such as cytotoxicity and high immunogenicity.

On the other hand, since negative-sense RNA viruses, such as Sendai virus, are not integrated into chromosomes, they do not activate cancer genes. Furthermore, since Sendai virus is an RNA virus, it has advantages, such as protein expression in short time after infection and an extremely higher level expression of the transgene product compared with Adenovirus.

In Biosis Abstract PREV 199799322964 oligonucleotides complexed with hemagglutinating virus of Japan (HUJ) were delivered into cells of the CNS.

### Disclosure of the Invention

It is an objective of this invention to provide an in vitro method for transferring nucleic acid using a negative-sense RNA viral vector, useful for gene therapy of nerve cells.

The present inventors first prepared recombinant viruses carrying various foreign genes, using Sendai virus, a typical negative-sense RNA virus and useful as a vector for gene therapy because of its safety and convenience. Subsequently, these recombinants were used to transfer the foreign genes into nerve cells, brain tissues, etc. As a result, the inventors found that the use of these recombinants enabled an efficient transfer of foreign genes into nerve cells and brain tissues. Furthermore, they found that the use of viral vectors of this invention led to high level expression of foreign genes introduced.

In addition, viral vectors of this invention transferred into the brain exhibited the limited proliferation. In other words, the expression of the vectors was reduced after a certain period of foreign gene expression. Furthermore, the gene therapy using a viral vector of this invention was applied to the brain of a β-glucuronidase-deficient mouse, which improved the symptoms of said mouse. Thus, the present inventors discovered that the viral vectors prepared could efficiently function in gene therapy of neuropathy where the therapy requires regulation of transgene expression.

The intraventricular administration of a viral vector of this invention carrying an FGF gene to jirds or mice resulted in the vector infection of ependymal cells and the decrease of the food intake and body weight in the animals. Ependymal cells form a cell layer that separates the brain from ventricles, and in the third ventricle the cerebrospinal fluid and hypothalamic nuclei intimately interact. Since vectors of this invention can efficiently infect ependymal cells, they can be used to express a secretory protein in the ventricle so that the protein acts on hypothalamic nuclei (feeding center, satiety center, etc.). In addition, in an ischemic model using jirds, it has been revealed that the cell injury is significantly reduced by introducing a viral vector for a growth factor expression into the hippocampus parenchymal cells, indicating a usefulness of the vector of this invention for preventing the cell death due to cell exfoliation in brain ischemia. These facts have indicated that vectors of this invention are useful as vectors for transfer of gene into the brain in various medical treatments.

The present invention relates to:
1. A method for transferring nucleic acid into nerve cells *in vitro,* comprising a step of contacting the nerve cells with a negative-sense RNA viral vector comprising a foreign gene in its genome or contracting the nerve cells with cells comprising said vector, wherein said negative-sense RNA virus belongs to the Paramyxoviridae family;
2. Use of a negative-sense RNA viral vector for the manufacture of a medicament for controlling the feeding behavior of animals, wherein said vector comprises a foreign gene encoding fibroblast growth factor (FGF)-1 or FGF-5 in its genome, and said negative-sense RNA virus belongs to the Paramyxoviridae family;
3. Use of a negative-sense RNA viral vector for the manufacture of a medicament for nerve cell-targeted gene therapy, wherein said vector comprises a foreign gene in its genome, and said negative-sense RNA virus belongs to the Paramyxoviridae family;
4. Use of 3, wherein said nerve cells are the central nervous system cells;
5. Use of 4, wherein said central nervous system cells are ventricular ependymal cells,
6. Use of 4, wherein said central nervous system cells are hippocampus cells,
7. Use of any one of 2 to 6, further comprising allowing to transiently express said foreign gene;
8. Use of any one of 2 to 7, wherein said foreign gene encodes a secretory protein;
9. Use of 8, wherein said protein acts on the hypothalamic nuclei;
10. Use of 8, wherein said protein is capable of protecting the brain from ischemia;
11. Use of any one of 2 to 10, wherein said virus belonging to the Paramyxoviridae family is Sendai virus; and
12. A negative-sense RNA viral vector for the in vivo gene therapy of nerve cells, said vector comprising a foreign gene encoding a secretory protein in its genome, wherein said negative-sense RNA virus belongs to the Paramyxoviridae family.

In this invention, "negative-sense RNA viral vectors" include a complex that is derived from a negative-sense RNA virus and has the infectivity. Herein, "infectivity" means the "capability of a complex to transfer its nucleic acid or other substances inside thereof into a cell through its ability to adhere and fuse to the cell membrane".

In this invention, a negative-sense RNA viral vector can be prepared by using, for example, a negative-sense RNA virus as a starting material. Viruses used as starting materials are viruses belonging to the Paramyxoviridae such as Sendai virus, Newcastle disease virus, mumps virus, measles virus, RS virus (Respiratory syncytial virus), rinderpest virus and distemper virus.

When Sendai virus is used, a group of proteins encoded by three genes, NP, P/C and L, which are thought to be essential for its autonomous replication, are not necessarily required to be encoded by the viral vectors of this invention. For example, the vector of this invention can be produced in the host cells that carry the genes encoding this group of proteins so that these proteins are provided by the host cells. In addition, the amino acid sequences of these proteins are not necessarily identical to those native to the virus. Any mutations can be introduced, or substitutions by homologous genes from other viruses can be used as long as their nucleic acid-transferring activities are equal to or higher than those of the naturally occurring proteins.

Further, when Sendai virus is used, a group of proteins encoded by the M, F and HN genes, which are thought to be essential for the disseminative capability of the virus, are not necessarily required to be encoded by the viral vectors of this invention. For example, the vector of this invention can be produced in the host cells that carry the genes encoding this group of proteins so that these proteins are provided by the host cells. In addition, the amino acid sequences of these proteins are not necessarily identical to those are native to the virus. Any mutations can be introduced into the genes or substitution of the genes by homologous gene from other virus can be used as long as their nucleic acid transferring activities are equal to or higher than that of the naturally occurring proteins.

To transfer a foreign gene into nerve cells, a complex comprising a recombinant viral genome into which a foreign gene is inserted can be prepared and used. The complex comprising a recombinant viral genome can be obtained by means of *in vitro* or *in vivo* transcription of a modified cDNA derived from any of the aforementioned viruses or a recombinant virus thereof followed by reconstitution of the virus. A method for reconstituting a virus has already been developed (see WO97/16539).

In addition, instead of the complete Sendai virus genome, incomplete viruses such as defective interfering particles (DI particles) (J. Virol. 68, 8413-8417, 1994), synthetic oligonucleotides, etc. may also be used as the component to constitute the complex.

When Sendai virus is used as a material, a complex may contain all the three genes, M, F and HF, which are involved in the disseminative capability of the virus. However, in general, even though a complex comprising all the M, F and HN genes is transferred into the brain, the complex presumably fails to exhibit disseminative capability after formation of the viral particles, because of the absence of protease to cleave F protein, a protein essential for the disseminative capability of Sendai virus. Herein, "disseminative capability" means "the ability of nucleic acid, which is transferred into a cell by infection or by employing an artificial technique, to replicate and direct the formation of infectious particles or their equivalent complexes which can disseminate the nucleic acid to other cells". However, to increase the safety, the genes involved in the disseminative capability of the virus are preferably eliminated or functionally inactivated in the viral genome in the complex. In the case of Sendai virus, genes involved in the disseminative capability of the virus are the M, F and/or HN genes. A reconstitution system of such complexes has been developed (WO97/16538). For example, for Sendai virus, a viral vector comprising a genome from which the F and /or HN genes are deleted can be prepared from the viral genome contained in the reconstituted complex. Such vectors are also included in the vectors of this invention for transferring nucleic acid into nerve cells.

The complex may contain on its envelope surface a factor that is capable of adhering to a specific cell, such as an adhesion factor, ligand, receptor, etc. For example, parts of the genes of a recombinant negative-sense RNA virus can be modified to inactivate the genes related to immunogenicity or to enhance the efficiencies of transcription and replication of RNA.

RNA contained in the complex can incorporate a foreign gene at its appropriate site. To express a desired protein, a foreign gene encoding the protein is incorporated into the RNA. For the Sendai virus RNA, a nucleotide sequence consisting of nucleotides in multiples of six is desirably inserted between the R1 and R2 sequences (Journal of Virology, 1993, Vol. 67, No. 8, pp. 4822-4830). Expression of the foreign gene inserted into the RNA can be regulated via the insertion site of the gene or the RNA sequence in the vicinity of the inserted gene. For example, in the case of Sendai viral RNA, it is known that the nearer to the NP gene the insertion position of the RNA comes, the higher the expression level of the inserted gene becomes.

A foreign gene encoded by the RNA contained in the complex can be expressed by infecting cells with the complex. As shown in the examples below, it has been demonstrated that a complex prepared as one embodiment of this invention by using the reconstitution system of Sendai virus enables an efficient transfer of a foreign gene into various nerve cell strains. As shown in Example 5, it has also been revealed that another embodiment of the complex of this invention in which the β-glucuronidase gene is used as a foreign gene shows a significantly higher expression level than retroviral vectors. Owing to these characteristics, the complex of this invention can be used for transferring genes into nerve cells. Since, one embodiment of the complex of this invention shown in Example 6 decreases its expression about one week after the intraventricular administration, it is useful in such a gene therapy that requires the gene expression of only for a limited period of time.

Nucleic acid or other compounds contained in the complex prepared can be introduced into nerve cells by contacting the complex with nerve cells or by directly contacting the viral vector-producing cells with nerve cells. When the complex is administered into the brain, the administration can be performed, for example, by boring a hole on the cranial bone after craniotomy under anesthesia, followed by injecting the complex using a glass needle or the like material. The complex can contain foreign genes. Foreign genes may include any types of genes, such as the nerve cell-specific gene, apoptosis-suppressing gene, other genes for treating various type of diseases, etc. Such genes can take the forms of antisense DNA and ribozyme so as to inhibit the function of a specific gene.

For example, it has been revealed that the brain cell death in ischemic tissues does not occur soon after ischemia, but within several days after that (Neurosci. Lett. 1998, 240: 69-72). To prevent the brain cell death in such a case, a complex of this invention comprising a gene responsible for suppression of the cell death, such as bcl-2, etc. can be used. In fact, during the investigation whether administration of the vector of this invention could prevent the delayed exfoliation of fragile nerve cells due to depletion of nutrients caused by ischemia, it was revealed that administration of an FGF-1 expression vector could significantly prevent the cell exfoliation (Example 10). In addition, as demonstrated in Examples 6 and 8, the complex of this invention can transfer a foreign gene into ependymal cells and cells present along the ventricles via intraventricular administration. Use of a gene expressing a secretory protein as a foreign gene can diffuse the protein through the spinal fluid into the brain including the hippocampal area. As shown in Example 7, it is also possible to express a foreign gene in the pyramidal cells of the hippocampus by administering a complex of this invention into the cells. As shown in Examples 6 and 7, one embodiment of the complex of this invention was expressed in nerve cells even 13 days after the administration of the complex into the brain. No cell death was caused by the transfer of the complex. These results indicate the usefulness of the complex of this invention for the gene therapy of central nerves. For example, in Example 9, it was demonstrated that the intraventricular administration of an FGF expression vector could successfully control the amount of food intake and reduce the body weight. Body weight loss attributable to FGF-2 (Denton, D. A. et al. (1995) Physiol. Behav. 57 (4): 747-752) and reduction of the blood sugar level accompanied with the body weight loss (Stephens, T. W. et al. (1995) Nature 377 (6549): 430-532) were already reported, which coinsides with the results obtained in the present invention that the blood sugar level was reduced associated with the body weight loss.

Thus, the vectors of this invention provides a novel mode of vector administration targeting ependymal cells. In addition to ependymal cells, target cells include, but not limited to, cells present along the ventricles, cells in the hippocampal region, especially hippocampus pyramidal cells, neural stem cells, neural crest cells derived from mammalian embryos, etc. Genes that can be introduced include, but not limited to, those for fibroblast growth factors, nerve growth factors, apoptosis inhibitors, heat shock proteins, peroxidases, etc. Specific exemples of such genes include those for FGF-1 (J. Biol. Chem. 271 (47): 30263-30271, 1996), FGF-5 (Proc. Natl. Acad. Sci. U.S.A. 87 (20): 8022-8026, 1990), NGF (Nature, 302 (2): 538-540, 1983), CNTF (Nature, 357 (6): 502-504, 1992), BDNF (EMBO J., 9 (8): 2459-2464, 1990; Genomics, 10 (3): 558-568, 1991), GDNF (J. Neurosci. Res. 41 (2): 279-290, 1995), p35 (J. Virol. 61 (7): 2264-2272, 1987), CrmA (Proc. Natl. Acad. Sci. U.S.A. 83: 7698-7702, 1986), ILP (EMBO J., 15 (11): 2685-2694, 1996), bcl-2 (Oncogene., 4 (11): 1331-6, 1989), ORP 150 (Biochem. Biophys. Res. Commun. 230 (1): 94-99, 1997), etc. Vectors of this invention are useful for not only searching genes by using DNA chips and DNA arrays, but also conveniently preparing model mice as well as developing medicines.

Animals into which the complex of this invention can be introduced include all kinds of mammals such as human, jird, mouse, rat, rabbit, cattle, monkey, etc.

### Brief Description of the Drawings

Figure 1 schematically shows a method for constructing the replication competent Sendai virus (SeV) comprising a foreign gene, such as for GFP or β-glucuronidase. Using primer 1, which has a NotI site, and primer 2, which comprises, a transcription termination signal (R2), an intervening sequence (IG), a transcription initiation sequence (R1) and a NotI site, the ORF of a foreign gene is amplified by PCR and inserted into the NotI site of pUC18/T7HVJRz.DNA (+18).
Figure 2 is a frontal cross sectional view of the moue brain showing the expression of GFP in a mouse infected with Sendai virus comprising the GFP gene (GFP-SeV).
Figure 3 is a cross sectional view of the lateral ventricle showing the expression of β-glucuronidase in a β-glucuronidase-deficient mouse 3 days after the infection with Sendai virus carrying the β-glucuronidase gene.
Figure 4A shows a cross sectional view of the lateral ventricle showing the β-glucuronidase expression (framed areas) in the ventricle of a β-glucuronidase-deficient mouse 12 days after the infection with Sendai virus carrying the β-glucuronidase gene. Figure 4B shows the section adjacent to that of Figure 4A stained by Lorbacher method.
Figure 5 is a graph showing changes in the body weight of jirds after the intraventricular administration of Sendai virus expressing FGF-1, FGF-5 and GFP.
Figure 6 is a graph showing changes in the body weight of mice after the intraventricular administration of Sendai virus expressing FGF-1, FGF-5 and GFP.
Figure 7 is a graph showing changes in the amount of food intake of mice after the intraventricular administration of Sendai viruses expressing FGF-1, FGF-5 and GFP.
Figure 8 is micrographs showing the delayed exfoliation of pyramidal cells in the hippocampal CA1 area of a jird 5 days after ischemia.
Figure 9 is micrographs showing the prevention of delayed exfoliation of pyramidal cells in hippocampal CA1 region after the administration of a FGF-1 expressing Sendai viral vector.

### Best Mode for Carrying out the Invention

The present invention will be explained in detail with reference to examples below, but is not to be construed as being limited thereto.

### Example 1. Preparation of the replication competent Sendai virus (SeV)

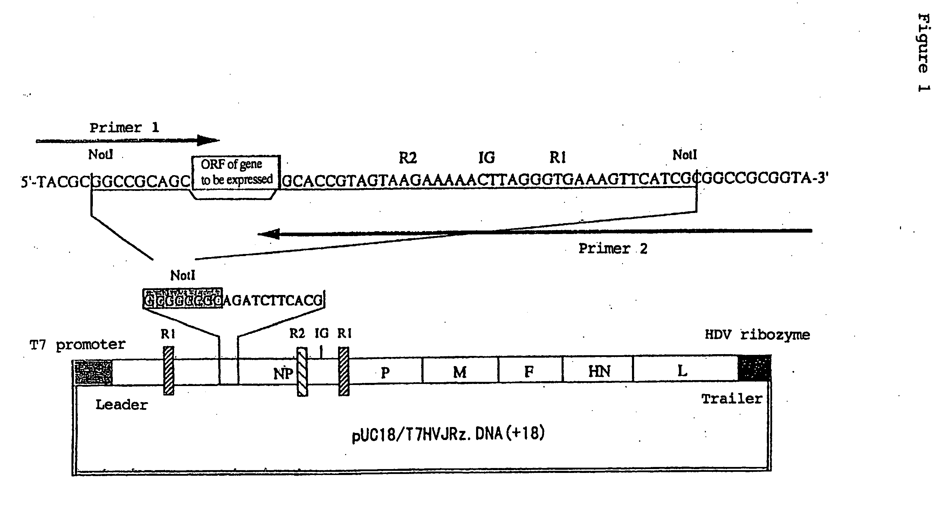

A NotI fragment comprising a foreign gene to be transferred, transcription initiation (R1) and termination (R2) signals, and intervening sequence (IG) (Fig. 1) was amplified by PCR and inserted into the NotI cleavage site of SeV transcription unit pUC18/T7HVJRz.DNA (+18) (Genes Cells, 1996, 1: 569-579) (Fig. 1). According to an established method (Genes Cells, 1996, 1: 569-579), using LLCMK2 cells and embryonated chicken eggs, the virus comprising the above-described genes was reconstituted, resulting in the recovery of the virus comprising the desired gene.

### Example 2. Confirmation of infectivity of "GFP-SeV" to established nerve cell lines

As the established cell lines, rat phenochromocytoma (PC12), human neuroblastoma (IMR-32) and human glioblastoma cells (A172) were used. PC12 cells were cultured in a DMEM medium supplemented with horse serum and calf serum to a final concentration of 5% for each serum. To promote neurite outgrowth, a nerve growth factor (NGF7S) was added to the medium to a final concentration of 50 ng/ml. An MEM medium containing 10% calf serum supplemented with an MEM sodium pyruvate solution and MEM non-essential amino acid solution to the final concentrations of 1 mM and 0.1 mM, respectively, was used for the culture of human neuroblastoma cells (IMR-32). Human glioblastoma cells (A172) were cultured in a MEM medium (a high glucose medium) containing 10% calf serum.

10⁵ cells were plated into a 6-cm dish containing NGF in the medium, incubated for 3 days to induce the neurite outgrowth and then used for PC12 cell infection experiment. After removing the medium, the cells were washed once with PBS. SeV into which a GFP gene is introduced (hereinafter referred to as GFP-SeV) was diluted with 500 µl of PBS supplemented with 1% bovine serum albumin to 10⁶ plaque forming unit (p.f.u.), and was added to the cells to infect GFP-SeV for 20 min under the conditions where the cells were protected from drying. After the infection, the medium (5 ml) was added to the plates, and the cells were cultured for 2 days. After culturing, the cells were examined for GFP fluorescence under a fluorescence stereoscopic microscope. As a result, the infection of PC12 cells with GFP-SeV was confirmed by the GFP fluorescence within the cells. Fluorescence emission could not be observed with the control cells infected with SeV carrying no GFP gene and non-infected cells.

IMR-32 cells (3 x 10⁵ cells) were plated into a 10-cm plate containing a predetermined medium, and cultured overnight. Based on the cell number estimated to be 6 x 10⁵ after the culture, GFP-SeV was diluted to m.o.i. (multiplicity of infection) of 10 with 1000 µl of PBS containing 1% bovine serum albumin. After the cells were infected with the virus for 20 min, they were cultured in a predetermined medium for 12 or 36 h, and then examined for the GFP fluorescence under a fluorescence stereoscopic microscope. After the culture for 12 h, fluorescence was observed in the cell body of GFP-SeV-infected cells. After the 36-h culture, GFP fluorescence was observed in the neurite portion in addition to the cell body. Fluorescence was not observed in the control cells infected with SeV carrying no GFP gene as well as non-infected cells.

A172 cells were also infected with the virus in a similar manner as that used for IMR-32 cells. Fluorescence was observed in the cell body of GFP-SeV-infected cells, but not in the control cells infected with SeV carrying no GFP gene as well as non-infected cells.

GFP-SeV infected all the established nerve cell strains used in the present study, and succeeded in expressing GFP from the GFP gene within the cells. These results indicated a possibility of the SeV infection of the primary culture of brain cells and of brain cells by *in vivo* administration of the virus.

### Example 3. Culture of the primary rat brain cells

An SD rat of 18-day pregnancy was deeply anesthetized with diethyl ether, and euthanized by the exsanguination from the axillary artery. After the abdominal region was disinfected with 95% ethanol, it was subjected to laparotomy to remove the fetuses together with the womb. Subsequent procedures were all performed under the germfree conditions on ice, or in ice-cold solutions unless otherwise stated. Fetuses was removed from the womb using scissors and roundheaded forceps, and transferred to a plate containing 20 ml of an operation solution (50% DMEM and 50% PBS). After the fetuses were placed on a sterilized gauze pad, their scalp and skull were incised along the midline using two pairs of INOX#4 forceps. Subsequently, a pair of INOX#7 forceps was inserted along the undersurface of the brain tissue to scoop up the brain tissue as a whole with the medulla oblongata being cut off, and the tissue was excised and placed in the operation solution. Under a stereoscopic microscope, the brain in the operation solution was filleted into three portions using two scalpels to separate the brain stem, and two pieces of cerebral hemispheres containing the hippocampus and corpus striatum were transferred into another operation solution with roundheaded forceps. Under a stereoscopic microscope, the meninx was completely removed from the surface of the brain tissue using two pairs of INOX#5 forceps, and transferred into another operation solution using roundheaded forceps for washing. Six pieces of cerebral hemispheres were placed into a preservation solution (90% DMEM (containing 5% horse serum and 5% calf serum), and 10% DMSO) with roundheaded forceps, and then they were cut into small pieces less than 1 mm using a scalpel on slides. The tissue pieces thus cut were placed into about 1.5 ml of the preservation solution in a pre-cooled tube, which was stored in a freezing container, frozen slowly over a period of 3 hours, and then stored in liquid nitrogen. The tissue pieces of 6 cerebral hemispheres were taken out from the liquid nitrogen, thawed at 32°C, washed twice in 8 ml of the operation solution, and allowed to stand for 30 sec, and then the supernatant was removed. To the tissue pieces were added 5 ml of an ice-cold papain solution (papain 1.5 U, cysteine 0.2 mg, bovine serum albumin 0.2 mg, glucose 5 mg, and DNase 0.1 mg/ml) which had been filtered and sterilized. The mixture was warmed at 32°C for 15 min and mixed by inverting the tube every 5 min. The supernatant was separated, and 5 ml of a solution containing 20% calf serum were added. A papain solution (5 ml) preheated to 32°C was added to the precipitate fraction, and the resulting mixture was further warmed for 15 min. The mixture was mixed by inverting the tube every 5 minutes. After good turbidity of the supernatant as well as translucence of the tissue pieces were confirmed, the tissue pieces were split by pipetting. The first supernatant fraction preheated to 32°C was added to this sample solution, and the resulting mixture was centrifuged in a centrifuge preheated to 32°C (at 1200 rpm for 5 min). After removal of the supernatant, 5 ml of DMEM (containing 5% horse serum and 5% calf serum) were added to and mixed with the residue to break the cells up, followed by centrifugation under the above-described conditions. After the removal of supernatant, 2 ml of DMEM (containing 5% horse serum and 5% calf serum) were added to the residue, and the resulting mixture was stirred. As a result of cell counting, the cell number was found to be 5 x 10⁶ cells/ml. The primary culture of brain cells thus obtained were seeded on a polyethylene imine-coated plate and cultured.

### Example 4. Confirmation of infectivity of SeV to the primary culture of brain cells using GFP-SeV

The primary culture of brain cells obtained in Example 3 was cultured in a 10-cm plate for 3 days. After the removal of the supernatant, a sample solution prepared by diluting GFP-SeV in 1000 µl of PBS containing 1% bovine serum albumin was added to the culture to infect with the virus for 20 min. After the infection, 10 ml of DMEM medium (containing 5% horse serum and 5% calf serum) was added, and the cells were cultured for 2 days. The cells were then examined for the fluorescence of GFP under a fluorescence stereoscopic microscope. Almost all the cells displayed fluorescence. That is, it was confirmed that SeV infects even the primary culture of brain cells.

### Example 5. Infection of Sendai virus carrying the β-glucuronidase gene (hereinafter abbreviated as β-glu-SeV) to human fibroblast cells deficient of the β-glucuronidase gene and expression of said enzyme in the cells

For the implementation of this invention, human fibroblast cells deficient of the β-glucuronidase gene (hereinafter abbreviated as β-glu-deficient cell) and human normal fibroblast cells were used.

Mucopolysaccharidosis type VII, one type of mucopolysaccharidosis, is caused by deficiency of β-glucuronidase, and shows a variety of clinical symptoms ranging from a mild case to severe case with fetal hydrops. There are many severe cases showing various symptoms developed during the infantile period, including characteristic facial feature, splenohepatomegary, psychcomotor retardation, bone deformation, etc.

It has been indicated that, for the intracellular transport of β-glucuronidase to lysosome, the addition of sugar chain to the enzyme molecule and the phosphorylation of the 6-position of the mannose moiety of the enzyme are necessary. On the arrival at lysosome, C-terminus of the enzyme undergoes proteolysis..

Prior to the implementation of this invention, β-glu-SeV was examined for 1) its infectivity to human fibroblast cells, 2) its expression amount, and 3) the presence of its molecular species to be transported to lysosome.
1) β-glu deficient fibroblast cells were prepared so that 10⁵ cells/well were placed in a 6-well plate. β-glu-SeV was diluted in 100 µl of PBS containing 1% bovine serum albumin so that the multiplicity of infection (m.o.i.) became 5, and the overnight-cultured β-glu deficient cells were infected for 1 hour. The cells were cultured in a serum-free MEM medium for 24 h. The cells thus cultured were fixed in a mixture of formalin and acetone (1:7, v/v). With naphthol AS-BI glucuronide as a substrate, the reaction was performed in the acetate buffer, pH 5.0, at 37°C, and the substrate decomposition was monitored by the red coloration. As a result, the cytoplasm of β-glu deficient cells incubated with "β-glu-SeV" was stained red, indicating that β-glu deficient cells were infected with "β-glu-SeV" to express the transferred gene.
2) β-glu deficient cells were prepared so that 10⁵ cells/well were placed in a 6-well plate. "β-glu-SeV" was diluted in 100 µl of PBS containing 1% bovine serum albumin so that the multiplicity of infection (m.o.i.) became 0.1 and 1.0, and incubated with overnight-cultured β-glu deficient cells for 1 h. The cells were cultured in a serum-free MEM medium for 24 or 48 h. After the incubation for the predetermined period of times, cells were recovered and sonicated to prepare intracellular fractions. With 4-methylumbelliferyl-β-D-glucuronide as a substrate, the amount of 4-methylumbelliferone (MU), the enzymatic reaction product, was determined by measuring the fluorescence intensity with a fluorospectrophotometer. The results are shown in Table 1. In this table, the expression amount was represented by the amount of 4-methylumbelliferone (MU) produced by 1 mg of protein in the intracellular fraction in 1 hour.

**Table 1**

| Cell | Infecting condition | Amount of expression (nmol MU/mg total protein/h) |
|---|---|---|
| β-glu-deficient fibroblast | No infection | 53 |
| Normal fibroblast | No infection | 276 |
| β-glu-deficient fibroblast | β-glu-retro | 911 |
| β-glu-deficient fibroblast | β-glu-SeV (m.o.i. = 0.1, 24 h) | 15,900 |
| β-glu-deficient fibroblast | β-glu-SeV (m.o.i. = 1.0, 24 h) | 27,100 |
| β-glu-deficient fibroblast | β-glu-SeV (m.o.i. = 0.1, 24 h) | 21,100 |
| β-glu-deficient fibroblast | β-glu-SeV (m.o.i. =1.0, 24 h) | 32,300 |

As shown in Table 1, the expression amount ranged 15,900-32,300 (nmol MU/mg total protein/h), and 276 for normal fibroblast cells and 911 for the cell expressing β-glucuronidase with a retrovirus (β-glu-retro), indicating that SeV strongly expresses a transgene in the SeV-infected cells.
3) The fractions obtained in 2) were used as the intracellular fraction of "β-glu-SeV"-infected-β-glucuronidase-deficient-fibroblast cells. As the culture supernatant fraction, proteins contained in the culture supernatant were recovered by precipitation with cold acetone. Test samples thus obtained were subjected to Western blot analysis using an anti-human β-glucuronidase antibody. As a result, in the intracellular fraction of "β-glu-SeV"-infected-β-glucuronidase-deficient-fibroblast cells, two types of proteins were identified; one has high molecular weight and another has low molecular weight, and both are reactive with the anti-human β-glucuronidase antibody. The band of the low molecular weight protein corresponds to that of the protein reactive with the anti-human β-glucuronidase antibody in normal fibroblast cells, indicating that it is a molecular species of β-glucuronidase the C-terminus of which has undergone proteolysis after transported to lysosome. The high molecular weight protein was not observed in the normal fibroblast cell, but present in the intracellular and supernatant fractions of β-glu-SeV-infected-β-glucuronidase-deficient-fibroblast cells. The supernatant fraction contained only the high molecular weight protein. This may be due to too high an expression of β-glucuronidase caused by β-glu-SeV infection, in which transport of the high molecular weight protein species to lysosome failed to catch up with such a high enzyme expression, resulting in the secretion of the protein into microsomes or extracellular space. Alternatively, judging from its molecular weight, the high molecular weight protein may be a molecular species with a sugar chain attached but without the 6-position of mannnose moiety being phosphorylated so that it cannot be transported to lysosome.

Thus, the human β-glucuronidase, which is assumed to be transported to lysosome, was able to be expressed in the intracellular fraction of β-glu-SeV-infected-β-glucuronidase-deficient-fibroblast cells.

### Example 6. Expression of GFP in ependymal cells by intraventricular administration of GFP-SeV

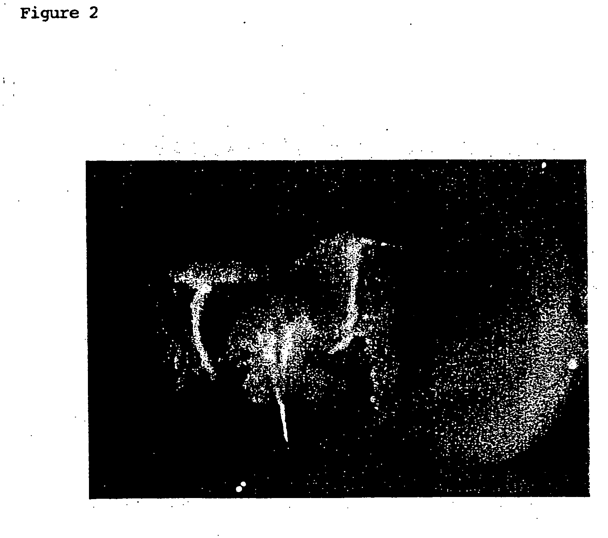

Mice of 8-10 weeks old were anesthetized with 200 µl of 10-fold diluted Nembutal. After craniotomy, a hole of 1 mm in diameter was bored in the skull at the position 1.0 mm from the bregma and 1.5 mm to the right of the medline with a dental drill. After the removal of the dura, GFP-SeV was administered at the position 1.3 mm deep using a 27 G syringe needle. The dose of GFP-SeV was 20 to 30 µl, and the number of the virus contained in the sample solution was estimated 1 x 10⁷ p.f.u.to1.5 x 10⁷ p.f.u. Control mice were administered PBS or SeV carrying no GFP gene. Autopsy was performed 3, 5, 7 and 10 days after the administration. A whole brain was removed, and a frontal cross section was made. Under a stereoscopic fluorescence microscope, GFP fluorescence was observed. In the dissected brain autopsyed 3 days after the administration of GFP-SeV, the conspicuous GFP fluorescence was observed. At the site along the ventricle of the frontal cross section, distinct fluorescence of GFP was observed (Fig. 2). As described in Example 8 below, SeV-infected cells emitting GFP fluorescence were thought to be ependymal cells. The cells along the lateral ventricle also became fluorescent 5 and 7 days after the infection. However, the fluorescence intensity was significantly decreased in the cells 7 days after the infection, and no fluorescent brain cells could be observed 10 days later. Fluorescence could not be observed in the control mouse brains to which PBS or SeV carrying no GFP gene had been administered as a control.

### Example 7. Administration of GFP-SeV to brain parenchyma under stereotaxy

To examine the SeV infection of nerve cells, especially pyramidal cells of hippocampus, which is the main object of this invention, precisely targeted administration of SeV to the vicinity of hippocampus is required. Therefore, a stereotaxy was conducted to introduce SeV into the brain parenchymal and the brain parenchyma cells were examined for the infection. As the experimental animals, 1) mouse and 2) rat were used.
1) Two holes of 1 mm in diameter each were bored through the skull at the position 2 mm to the left and right of the medline and 3 mm anterior to the bregma using a dental drill. GFP-SeV (1.5 µl each ) was administered to the parenchymal portions, 3.5 mm deep on the right side and 2.5 mm deep on the left side, using a glass capillary. The skull was closed, and surgically opened 3 days later to examine the GFP expression, which was observed in the parenchymal portion. After the fixation with ethanol, frozen tissue slices were prepared. Although GFP fluorescence was significantly reduced in the frozen slices after ethanol fixation due to the outflow of chromophores, fluorescent sites were still observed. In the white matter near the internal capsule, GFP fluorescence was observed on the axon from which myelin protein was eluted with ethanol. Furthermore, GFP fluorescence was also observed in the axon in the area presumed to be the corpus striatum.
   These results demonstrated that GFP-SeV was capable of infecting nerve cells of the mouse brain.
2) Since a precise stereograph has already been made for rat, GFP-SeV can be accurately administered to the vicinity of pyramidal cells in the hippocampus CA1 area. A rat weighing about 170 g was anesthetized, and, after craniotomy, two holes of 1 mm in diameter each were bored through the skull at the positions 2 mm to the left and right of the medline and 4.5 mm anterior to the internal (sigma) with a dental drill. GFP-SeV (1.5 µl each) was administered to the parenchymal portions, 3.5 mm deep on the right side and 2.5 mm deep on the left side, using a glass capillary. The skull was closed, and surgically opened 3 days later to examine the GFP expression. As a result, the GFP expression was observed in the hippocampus CA1 pyramidal cell area, where GFP-SeV was administered in 2.5 mm deep. Enlarged view of the region adjacent to the hippocampus by fluorescence microscopy revealed the marked fluorescence in the cell bodies of the hippocampus CA1 pyramidal cells and dendrites. The GFP expression was observed even in the pyramidal cells 13 days after the administration. Even 13 days after the administration of GFP-SeV, the GFP expression was observed in the cell bodies and dendrites of the pyramidal cells. These results demonstrate that SeV infection does not cause the nerve cell death even 13 days after the infection, strongly suggesting the usefulness of Sev as a vector for the gene therapy directed to prevention of the exfoliated cell death following the brain ischemia.

### Example 8. Gene therapy trial on β-glucuronidase-deficient mice using β-glu-SeV

The results of Example 6 indicates that the ependymal cells are infected with SeV by intraventricular administration. Therefore, the inventors conducted an experiment in which β-glu-SeV is administered to β-glucuronidase-deficient mice (J. Clin. Invest., 1989, 83: 1258-1266) to induce secretion of β-glucuronidase from the infected cells into the cerebrospinal fluid and then to be taken up by target cells so that the symptoms would be improved.

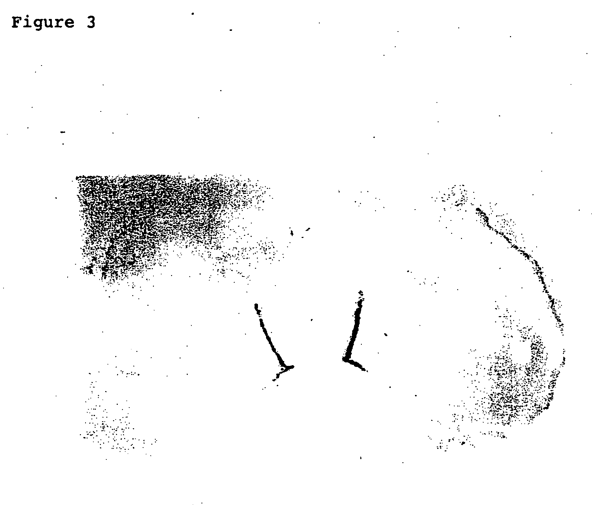

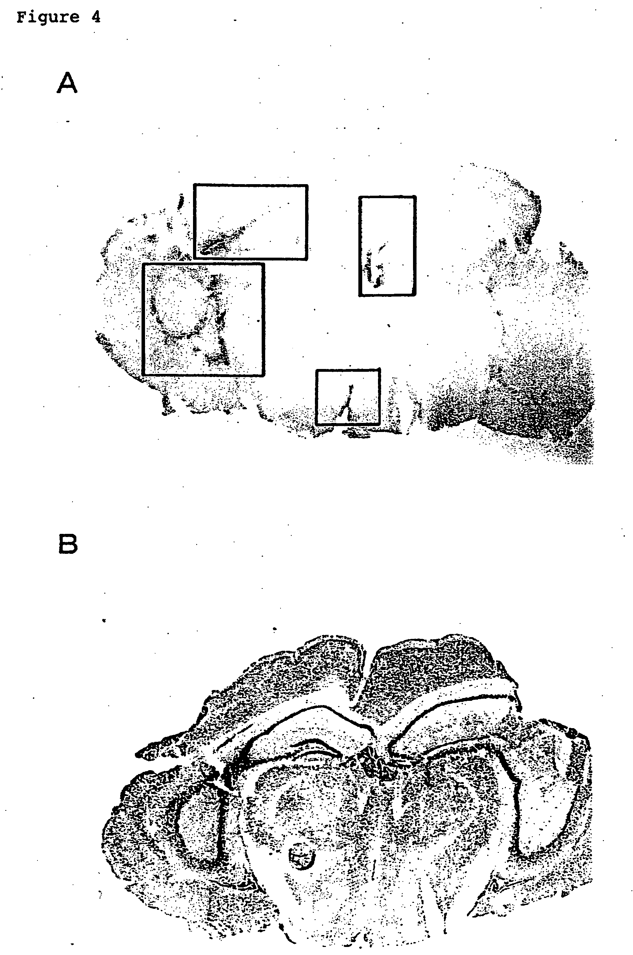

Homozygous mice were selected from mice obtained by breeding heterozygous mice based on the β-glucuronidase activity in the tail vein blood of the mice and on the presence of the Nlal IV cleavage site in the PCR amplification fragments of the β-glucuronidase gene-deficient site on the chromosomes of the mice, and were used in the present experiment. Administration of β-glu-SeV was carried out according to the method described in Example 6. The brain was excised 3 or 12 days after the administration to prepare the frozen tissue slices. The β-glucuronidase activity in the tissue was assayed using a modification of the method described in Example 5, 1). As shown in Figure 3, the sites at which β-glucuronidase was expressed were strongly stained red along the ventricles. When magnified by microscopy, the ependymal cells of the lateral ventricle were verified to strongly express β-glucuronidase, which was then secreted from the cells. On the tissue slice prepared 12 days after the administration (Figure 4), β-glucuronidase that had been expressed in and then secreted from the ependymal cells of the lateral ventricle was shown to be diffused into the ventricle with the migration of the spinal fluid to reach the vicinity of the hippocampus. Physical capabilities of the homozygous mice was apparently improved, although slightly, by this administration.

### Example 9. Experiments on eating depression caused by administration of the Sendai viral vector carrying FGF-1 or -5 (eating depression experiments in jirds and mice)

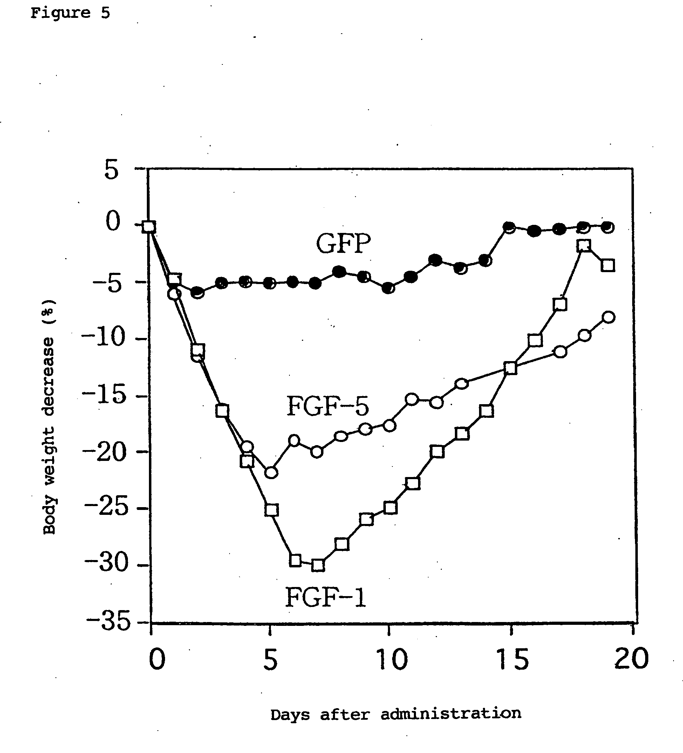

Jirds (weighing 60 to 80 g) were anesthetized with Nembutal, fixed to a stereotactic instrument, depilated, and then incised in the scalp along the medline. A hole was bored in the skull at the position 1.0 mm from the bregma and 1.5 mm to the right of the medline using a dental drill with care to avoid damaging the blood vessels under the cranial bone. After drilling the hole, the dura and others were removed with tweezers. Mouse FGF-1-SeV (5 x 10⁶ pfu), human FGF-5-SeV (1 x 10⁷ pfu) and GFP-SeV (5 x 10⁶ pfu) were injected 1.0 mm deep into the right lateral ventricle (n = 2) with a 30G syringe needle. The recombinant viruses were prepared according to Example 1. Changes in the body weight were monitored by measuring the weight, and decrease in the body weight was observed from the next day of the administration (Figure 5). In the FGF-1-administered group, the body weight started to decrease from the next day of the administration, and continued to decrease by about 5% everyday till 5 days later, resulting in a 29.5% decrease 6 days later, and the maximum decrease of 29.8% was observed 7 days later. Then, the body weight turned to increase, and was recovered to a 3.5% decrease 20 days after the administration. In the FGF-5 administered group, the body weight started to decrease from the next day, reached the maximum of 21.7% decrease 5 days after the administration, and then turned to increase, being recovered to a 8.0% decrease 20 days later. In the FGF-9 administered group, similar decrease in the body weight was observed from the next day, showed the maximum of 22.9% 5 days after the administration, and then turned to increase, being recovered to a 6.40% decrease 20 days later. In the control group to which GFP-SeV was administered, the maximum of a 5.8% decrease in the body weight, which was presumably caused by the administration itself, was observed. However, the rate of the body weight loss was relatively small as compared with the FGF-administered groups, clearly indicating that FGF affects the body weight loss.

Since the body weight decrease due to the administration of FGF-1-SeV and FGF-5-SeV was observed in jirds, more-detailed study was performed using B-6 mice (weighing about 20-22 g). The right lateral ventricle was selected as the administration site, and a hole of 1.0 mm in diameter was bored in the skull at the position 1.0 mm from the bregma and 1.5 mm to the right of the medline with a dental drill. After the removal of the dura, a sample was administered to the animal in the hole at the depth of 1.3 mm with a 27G-syringe needle. The sample solutions were prepared by adding 9 µl, 8 µl and 9 µl of PBS to 1 µl of FGF-1-SeV (1 x 10⁶ pfu), 2 µl of FGF-5-SeV (2 x 10⁶ pfu), and 1 µl of control GFP-SeV (1 x 10⁶ pfu) solutions, respectively. The body weight and the food intake were monitored for 2 weeks after the viral administration.

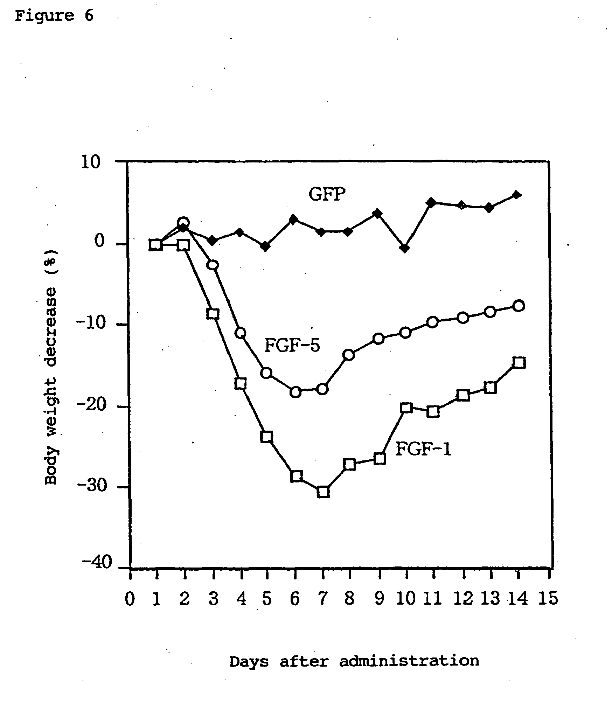

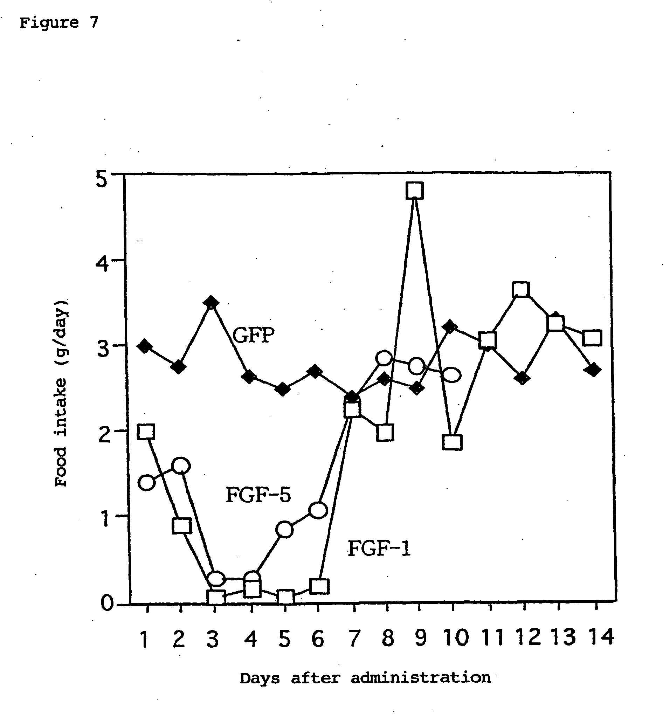

The control mice administered with GFP-SeV showed no decrease in the body weight, but showed a 7.5% increase as compared with the weight measured prior to the administration (Figure 6). The amount of the food intake was also not significantly changed (Figure 7). In the FGF-SeV-administered group, an average 30.5% decrease in the body weight was observed 6 days after the administration (Figure 6). Then, the body weight turned to increase, resulting in a 13.5% decrease weight 2 weeks later. The change in the amount of food intake due to the FGF-1 administration was so dramatic that almost no food intake was observed from day 2 to day 6, especially from day 3 to day 6 after the administration (Figure 7). In the FGF-5-SeV administered group, although the decrease in the body weight was also observed, the rate of decrease was smaller as compared with the FGF-1-SeV-administrated group, and a 17.9% decrease at the maximum (Figure 6). The effect on the body weight decrease was in a tendency similar to that obtained in the jird experimental system. Although the effect of the FGF-5-SeV administration on the body weight decrease was smaller than that of the FGF-1-SeV administration, the decrease in the food intake was clearly observed (Figure 7).

As shown in the results of the example, the effect of the intraventricular expression of FGF induced by SeV on the body weight decrease was a 30% decrease at the maximum. Considering that the effect of the intraventricular injection of FGF in the purified protein form on the decrease in the body weigh was 7 to 8% at most, the rate of 30% achieved in the present invention was shown to be extremely high. Difference in these effects may be due to the difference in the intraventricular accumulation of FGF depending on the administration methods, but there is another possibility that this difference is due to a direct action of FGF on nerve cells through the SeV infection to ependymal cells. As to the feeding control in the brain, only the control by the nerve nuclei of hypothalamus has been reported. In view of this, it is inferred that SeV efficiently infects ependymal cells to secrete a functional protein into the cerebrospinal fluid in the ventricle, and that said secretory protein efficiently acts on the hypothalamic nerve nuclei to exert the feeding control. This inference would be supported by the facts that a part of the hypothalamic nerve tissue has a nerve construction with the tight junctions of the blood-brain barrier being lost and contains neurons to receive liquid factors in the peripheral circulation and cerebrospinal fluid.

Among the hypothalamic nuclei, chemosensitive neurons are present in the ventromedial hypothalamus (VMH) and lateral hypothalamic area (LHA), which are thought to be the feeding and satiety centers, and the neuron activity alters in response to metabolic products and hormones contained in blood and cerebrospinal fluid. These VMH and LHA neurons to respond to glucose, and certain cytokines and growth factors are also known to function as appetite regulators. In addition, it has been demonstrated that, from the disruption experiment, the paraventricular nucleus (PVN) is also responsible for suppression of food intake. This nucleus has neurons that produce corticotropin releasing hormone (CRH) and shows the eating depression and activation of sympathetic nerve activity. Furthermore, the arcuate nucleus (ARC) is the site to produce NPY, a food intake stimulator, which is suggested to target PVN. The results of the experiments on the control of eating behavior described herein suggest that FGF acted on the nerve nuclei. Attention should be paid on the relation with leptin, which is expressed in mature adipocytes having lipid droplets and has been extensively studied in relation to eating behaviors as well as NPY, etc.

### Example 10. Experiment on suppression of ischemic cell exfoliation by using jirds

The area exposed to brain ischemia undergoes cell damage, and is further led to the cell death as the ischemia progresses. The extent of cell death depends on the degree and duration of ischemia. In the case of severe ischemia, not only nerve cells but also all constitutive cells in the ischemic area sustain irreversible injuries in a short period of time, resulting in the formation of brain infarction focus caused by necrosis. However, in the case of severe ischemic stress of short duration, or in the case of slight ischemia of long duration, the cells in the ischemic region become fragile depending on the severity of ischemia. The most fragile cells are nerve cells, and then oligodendrocytes follow. Astroglia, microglia, and vascular endothelial cell have been known to be more resistant to the ischemic stress. From the examination using a diffuse brain ischemia model, it has been known that there are differences in the resistance to ischemic stress among nerve cells. The known most fragile cells include nerve cells of the hippocampus CA1, those of the hilum of dentate gyrus, and those of the vestibular nuclei in the occipital region of head, which show a delayed cell death. The delayed nerve cell death is a good model of selective nerve cell death with high reproducibility independent of the energy insufficiency, contributing a great deal to the elucidation of molecular mechanisms of ischemic cell death. There have been many reports on the experiments using these model systems to examine, for example, what cascade the nerve cells may go through to their death, which step of the cascade is critical to protect the cell, into what type of cell death the delayed nerve cell death is classified, etc.

As the experimental model animals, rats, jirds and mice are often used. These animals are used to study and treat the pathologic changes in the portions vulnerable to ischemia, such as hippocampus, corpus striatum, etc., induced by causing transient ischemia in the whole brain of the ischemia modelsfor several to several ten minutes. A rat four vessel occlusion model, a rat hypotensive bilateral common carotid artery occlusion model, a bilateral common carotid artery occlusion model of jirds, etc. are frequently used as the ischemia model. The present inventors carried out an ischemia experiment using a bilateral common carotid artery occlusion model of jird. It has been known that in jirds cell death occurs mainly in most of the pyramidal cells in the hippocampal CA1 area when animals are subjected to a short time (5 min) ischemia. Therefore, the present inventors performed an experiment aiming at prevention of the cell exfoliation after ischemia by introducing into SeV a gene capable of preventing the cell death and administering the resulting complex to the hippocampus of jirds.

### <Preparation of an ischemic cell death model of jird>

Experiments were carried out with a bilateral common carotid artery occlusion (5 min) model of jird (gerbil species). By occluding (for 5 min) the bilateral common carotid artery of a jird, the pyramidal cells of hippocampus are selectively exfoliated 3-5 days after the occlusion. However, since this phenomenon is not commonly observed among jirds, it is necessary to screen jirds excellent as a model animal from those obtained from a commercial source. The jirds selected by the screening (obtained from Instructor Dr. Maeda, Department 1 of Anatomy, Osaka Municipal University) were used for the experiment.

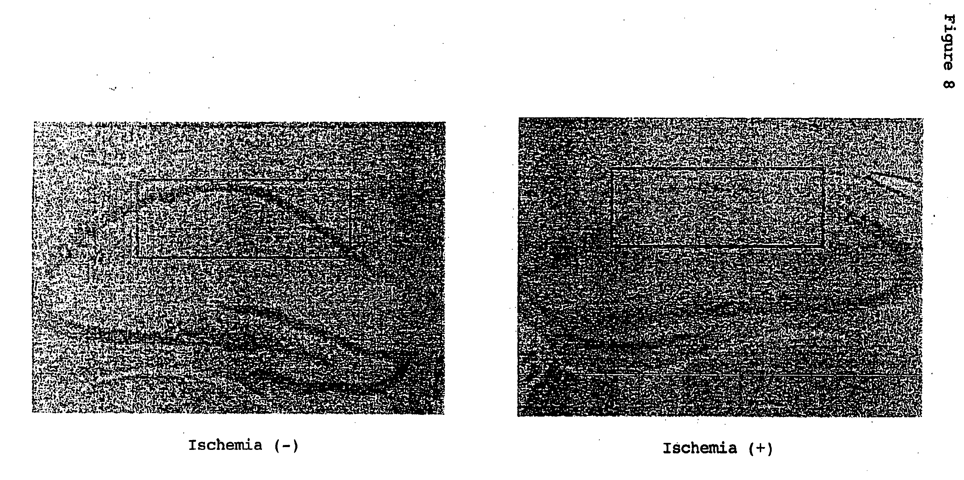

After anesthetized with ketamine, the animals were subjected to thoracotomy to find out the carotid arteries on the left and right sides of the trachea, and fat adhering to the carotid artery was removed. After the fat removal, the carotid arteries were occluded for 5 min with clips. During this procedure, since the rate of nerve cell death is significantly reduced when the brain and body temperatures are low, the animals were kept warm to retain the body temperature at 37.5°C being monitored with a thermometer inserted into the anus. The clips were removed 5 min later, and the blood was perfused again. Five days later, the jirds were sacrificed, and, after the craniotomy, the brain was excised to prepare tissue slices in paraffin. Conditions of nerve cells were confirmed by toluidine staining. As expected, the exfoliation of the pyramidal cells was observed in the hippocampal CA-1 area (Figure 8). Thus, the ischemic cell death model of jird has been prepared.

### <Experiment on prevention of nerve cell death by introduction of the recombinant SeV>

The SeV vector prepared above is used to examine whether the Sev vector is effective for preventing the nerve cell as follows: On the day before ischemia, the virus is introduced into only the right brain of the jirds. Ischemia is applied on the next day, and the animals are sacrificed 5-6 days later to observe the hippocampus pyramidal cells.

### <Transfer of FGF-1-SeV into hippocampus>

Jirds weighing 60-80 g were selected and used in this experiment. After anesthetized with Nembutal, the animals were fixed to a stereotactic instrument. The brain was then depilated and the scalp was cut open along the midline of the brain. A hole was bored through the skull at the position 5 mm from the bregma and 2 mm to the right of the midline using a dental drill with care not to damage the blood vessels under the cranial bone. After drilling the hole, the dura and others were removed with tweezers. An administration glass needle was inserted into the position at the depth of 1.4 mm, and the animals were allowed to stand for 2 min. Through the glass needle, 0.5 to 1.0 µl of an FGF-Sendai virus solution (virus of 1.0 x 10⁶ pfu to 2.0 x 10⁶ pfu) was injected to the position in a period of 12 min, and the animal was allowed to stand for further 10 min. The needle was removed, and the incision was sewed up. In this procedure, the virus was administered only to the right brain, and the exfoliation of nerve cells after ischemia was determined by comparing the right and left brains.

### <Ischemic operation>

After anesthetized with ketamine, the animals were subjected to thoracotomy to find out the carotid arteries on the left and right sides of the trachea, and fat adhering to the carotid arteries was removed. After the fat removal, the carotid arteries were occluded for 5 min with clips. During this procedure, since the nerve cell death is significantly reduced when the brain and body temperatures are low, the animals were kept warm to retain at the body temperature at 37.5°C, being monitored using a thermometer inserted into the anus. The clips were removed 5 min later, and the blood was perfused again. Five to six days later, the animals were sacrificed.

### <Preparation of paraffin sections>

After the animal was sacrificed, frontal cross sections of the hindbrain were made into 300-500 µm thick slices, soaked in 4% paraformaldehyde overnight, and embedded in paraffin with an automatic apparatus for fixation and embedding. The sections (5 µm thick) were prepared, deparaffinized, and subjected to immnohistochemical staining and other stainings.

### <Immunohistochemical staining>

Sections of the FGF-1-administered brain were prepared to examine for the reactivity to an antibody against the virus, to an anti-tubulin antibody (to determine the effect of ischemic operation), to an anti-GFAP antibody (to examine the astrocyte movement), and to an apoptag antibody (to examine the presence of apoptosis). The results are briefly summarized as follows (Table 2).

**Table 2**

| Determinations of the effect of FGF-1 | Antibody | Determination |
|---|---|---|
| Introduction of the virus into the hippocampal area | anti-virus antibody | ○ |
| Determination of the effect of the ischemic operation | Anti-b tubulin antibody | ○ |
| Morphology of the soma | HE staining | ○ |
| Movement of astrocytes | Anti-GFAP antibody | ○ |
| Presence of apoptosis | Apoptag | ○ |

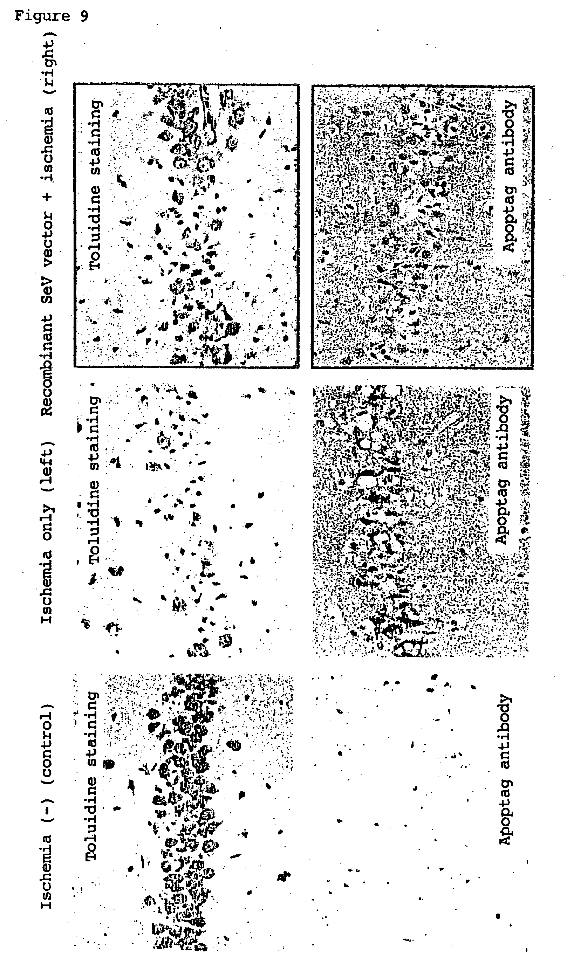

In the pyramidal cells of the hippocampal CA-1 region, HE staining did not reveal any changes in the nerve cells in the control sample, which underwent no ischemia. Many of the cells in one side of the brain which underwent ischemia but were not administered with the virus were atrophic nerve cells displaying nuclear condensation in the nucleus and eosinophilic change in the cytoplasm, so-called ischemic changes. In contrast, in the other side of the brain, which underwent ischemia and was administered with the virus, a small number of deformed nerve cells were observed to be dispersed, but a majority of the nerve cells retained the original morphology. On the side to which the virus was administered, a region that was positive for the antibody against the virus was observed. In the nerve cells that underwent ischemia but were not administered with the virus, the most of the cells that showed deformation were positive for the apoptag-staining. In contrast, in the cells which underwent ischemia and were administered with the virus, only a very few cells that stained with HE and showed the morphological change were positive for the apoptag-staining, indicating that apoptosis was suppressed in the majority of the cells in this side (Figure 9).

### Industrial Applicability

The present invention has provided an in vitro method for transferring a gene into nerve cells in the tissues including the central nervous tissue, into which transfer of a gene has hitherto been difficult. Subject matter related thereto is also provided. The invention enables the efficient transfer of a desired gene into the cells in gene therapy.

## Claims

1. A method for transferring nucleic acid into nerve cells *in vitro,* comprising a step of contacting the nerve cells with a negative-sense RNA viral vector comprising a foreign gene in its genome or contacting the nerve cells with cells comprising said vector, wherein said negative-sense RNA virus belongs to the Paramyxoviridae family.

2. Use of a negative-sense RNA viral vector for the manufacture of a medicament for controlling the feeding behavior of animals, wherein said vector comprises a foreign gene encoding fibroblast growth factor (FGF)-1 or FGF-5 in its genome, and said negative-sense RNA virus belongs to the Paramyxoviridae family.

3. Use of a negative-sense RNA viral vector for the manufacture of a medicament for nerve cell-targeted gene therapy, wherein said vector comprises a foreign gene in its genome, and said negative-sense RNA virus belongs to the Paramyxoviridae family.

4. Use of claim 3, wherein said nerve cells are the central nervous system cells.

5. Use of claim 4, wherein said central nervous system cells are ventricular ependymal cells.

6. Use of claim 4, wherein said central nervous system cells are hippocampus cells.

7. Use of any one of claims 2 to 6, further comprising allowing to transiently express said foreign gene.

8. Use of any one of claims 2 to 7, wherein said foreign gene encodes a secretory protein.

9. Use of claim 8, wherein said protein acts on the hypothalamic nuclei.

10. Use of claim 8, wherein said protein is capable of protecting the brain from ischemia.

11. Use of any one of the claims 2 to 10, wherein said virus belonging to the Paramyxoviridae family is Sendai virus.

12. A negative-sense RNA viral vector for the in vivo gene therapy of nerve cells, said vector comprising a foreign gene encoding a secretory protein in its genome, wherein said negative-sense RNA virus belongs to the Paramyxoviridae family.

## Patentansprüche

1. Verfahren zum Übertragen von Nukleinsäuren in Nervenzellen *in vitro,* umfassend einen Schritt eines Inkontaktbringens der Nervenzellen mit einem Virusvektor mit negativ-strängiger RNA, der ein Fremdgen in seinem Genom umfasst, oder Inkontaktbringen der Nervenzellen mit den Vektor umfassenden Zellen, wobei das Virus mit negativ-strängiger RNA zu der Familie der Paramyxoviridae gehört.

2. Verwendung eines Virusvektors mit negativ-strängiger RNA zur Herstellung eines Medikaments zum Steuern des Nahrungsaufnahme- bzw. Fressverhaltens von Tieren, wobei der Vektor ein Fremdgen umfasst, das in seinem Genom Fibroblasten Wachstums Faktor (FGF)-1 oder FGF-5 kodiert, wobei das Virus mit negativ-strängiger RNA zu der Familie der Paramyxoviridae gehört.

3. Verwendung eines Virusvektors mit negativ-strängiger RNA zur Herstellung eines Medikaments zur auf Nervenzellen zielgerichteten Gentherapie, wobei der Vektor ein Fremdgen in seinem Genom umfasst, und das Virus mit negativ-strängiger RNA zur Familie der Paramyxoviridae gehört.

4. Verwendung nach Anspruch 3, wobei die Nervenzellen Zellen des Zentralnervensystems sind.

5. Verwendung nach Anspruch 4, wobei die Zellen des Zentralnervensystems ventrikuläre Ependym-Zellen sind.

6. Verwendung nach Anspruch 4, wobei die Zellen des Zentralnervensystems Zellen des Hippocampus sind.

7. Verwendung nach einem der Ansprüche 2 bis 6, weiter umfassend, dass das Fremdgen vorübergehend exprimiert werden kann.

8. Verwendung nach Anspruch einem der Ansprüche 2 bis 7, wobei das Fremdgen ein sekretorisches Protein kodiert.

9. Verwendung nach Anspruch 8, wobei das Protein auf die Kerne des Hypothalamus wirkt.

10. Verwendung nach Anspruch 8, wobei das Protein das Gehirn vor einer Ischämie schützen kann.

11. Verwendung nach einem der Ansprüche 2 bis 10, wobei das Virus, das zu der Familie der Paramyxoviridae gehört, ein Sendaivirus ist.

12. Virusvektor mit negativ-strängiger RNA für die *in vivo* Gentherapie von Nervenzellen, wobei der Vektor ein Fremdgen umfasst, das ein sekretorisches Protein in seinem Genom kodiert, wobei das Virus mit negativ-strängiger RNA zu der Familie der Paramyxoviridae gehört.

## Revendications

1. Un procédé pour transférer *in vitro* de l'acide nucléique dans des cellules nerveuses comprenant une étape de contact des cellules nerveuses avec un vecteur viral à ARN à sens négatif comprenant un gène étranger dans son génome ou de contact des cellules nerveuses avec des cellules comprenant ledit vecteur, dans lequel ledit virus à ARN à sens négatif appartient à la famille des Paramyxoviridae.

2. Utilisation d'un vecteur viral à ARN à sens négatif pour la fabrication d'un médicament pour le contrôle du comportement alimentaire des animaux, dans laquelle ledit vecteur comprend dans son génome un gène étranger codant le facteur de croissance du fibroblaste (FGF)-1 ou le FGF-5 et ledit virus à ARN à sens négatif appartient à la famille des Paramyxoviridae.

3. Utilisation d'un vecteur viral à ARN à sens négatif pour la fabrication d'un médicament pour la thérapie génique ciblée sur des cellules nerveuses, dans laquelle ledit vecteur comprend un gène étranger dans son génome et ledit virus à ARN à sens négatif appartient à la famille des Paramyxoviridae.

4. Utilisation de la revendication 3 dans laquelle lesdites cellules nerveuses sont des cellules du système nerveux central.

5. Utilisation de la revendication 4 dans laquelle lesdites cellules du système nerveux central sont des cellules épendymes ventriculaires.

6. Utilisation de la revendication 4 dans laquelle lesdites cellules du système nerveux central sont des cellules de l'hippocampe.

7. Utilisation de l'une quelconque des revendication 2 à 6 comprenant en outre de permettre l'expression transitoire dudit gène étranger.

8. Utilisation de l'une quelconque des revendications 2 à 7 dans laquelle ledit gène étranger code une protéine sécrétoire.

9. Utilisation de la revendication 8 dans laquelle ladite protéine agit sur le noyau hypothalamique.

10. Utilisation de la revendication 8 dans laquelle ladite protéine est capable de protéger le cerveau de l'ischémie.

11. Utilisation de l'une quelconque des revendications 2 à 10 dans laquelle ledit virus appartenant à la famille des Paramyxoviridae est le virus de Sendai.

12. Un vecteur viral à ARN à sens négatif pour la thérapie génique *in vivo* de cellules nerveuses, ledit vecteur comprenant dans son génome un gène étranger codant une protéine sécrétoire, dans lequel ledit virus à ARN à sens négatif appartient à la famille des Paramyxoviridae.
